# EUROPEAN PATENT APPLICATION

(11) **EP 4 538 284 A1**
(43) Date of publication of application: **16.04.2025**
(21) Application number: 23203126.0
(22) Date of filing: 12.10.2023
(51) Int. Cl.: C07K 14/315, A61K 38/00, A61K 39/02, A61P 31/04

(54) **NOVEL ENGINEERED STREPTOLYSIN O DERIVED EPITOPES**

(71) Applicant: Tanea Medical AB, 753 20 Uppsala (SE)
(72) Inventor: MALMSTRÖM, Anders Johan, 221 84 Lund (SE); MALMSTRÖM, Lars Gustav, 221 84 Lund (SE); TANG, Di, 221 84 Lund (SE); HJORTSWANG, Elisabeth, 221 84 Lund (SE); GUETO TETTAY, Carlos, 221 84 Lund (SE)
(74) Representative: Zacco Denmark A/S

(57) **Abstract**

The present invention relates to immunology, antibody engineering, and vaccines. Specifically the present invention relates to Streptolysin O epitope variants having improved properties. More specifically, the present invention provides Streptolysin O epitope variants, immunogenic proteins, fusion proteins, protein nanoparticles and pharmaceutical compositions comprising these variants, as well as their use in the treatment of *S. pyogenes* associated diseases, disorders or conditions.

## Description

### Technical field of the invention

The present invention relates to the fields of immunology, vaccines and pharmaceuticals products. Specifically the invention relates to engineered epitopes, nanoparticles, antibodies, pharmaceutical compositions, bacterial vaccines and methods for preparing and using same.

### Background of the invention

Recent advancements in the characterization of the neutralizing antibody responses to human pathogens, when combined with protein engineering methods, have emerged as a promising strategy for designing vaccine candidates that elicits antibody responses of high magnitude and specific activity¹. The rational, antibody-guided design of vaccines relies on understanding the atomic structure of the antigen-antibody complexes to define protective epitopes as a starting point for reversed molecular engineering². Typically, structural biology methods such as nuclear magnetic resonance, X-ray crystallography and cryo-electron microscopy provide insights into the 3-D structure of antigens and the antigen-antibody complexes³. The recent development of structural mass spectrometry methods has emerged as an alternative strategy to define epitopes of relevance for immunity. Hydrogen deuterium exchange mass spectrometry (HDX-MS) has proven useful in the mapping of epitopes between monoclonal antibodies and single antigens^{4,5}. Additionally, recent studies have shown that epitope mapping can be accomplished by HDX-MS using polyclonal antibody mixtures, at least for small antigens⁶. Another approach is chemical cross-linking mass spectrometry (XL-MS), which facilitates the identification of proximal structural regions on amino acid level⁷. Protein samples are combined with reagents that form covalent cross-linked bonds in solution, and upon protein digestion, the resulting peptide pairs can be identified by tandem mass spectrometry⁸. The cross-linked peptide pairs generate distant constraints that can in conjunction with protein structural modeling resolve protein binding interfaces within antigen-antibody complexes^{,9,10}. This method is fast, does not require complex sample preparation protocols, necessitates relatively small amounts of starting material, can be applied to most proteins and is scalable to more complex mixtures of proteins, but does require knowledge of the primary amino acid sequences for the antibodies of interest.

Streptolysin O (SLO) is a 60 kDa pore-forming toxin ubiquitously produced by Streptococcus pyogenes (or Group A streptococcus, GAS) and belongs to a large protein family of cholesterol-dependent cytolysins (CDC). SLO features a disordered region of the first 69 N-terminal residues, followed by 3 discontinued Domains (D1-D3) and membrane-binding Domain (D4)¹¹. The primary function of SLO is to bind to cholesterol-rich membranes and induce cytolysis of eukaryotic cells. The cytolysis depends on a multi-stage process that starts with SLO binding to cholesterol-rich membranes, followed by oligomerization to form a pre-pore and conformational shifts in Domain 3 to span the membrane, leading ultimately to cytolysis¹². In addition to this main biological function, SLO also acts as an immune-modulatory molecule for neutrophils and impairs phagocytic clearance of GAS. Sub-cytotoxic levels of SLO have been found to suppress neutrophil oxidative bursts, enabling GAS to resist immediate cell killing¹³. SLO-mutant vaccines can counteract this inhibitory effect, although the protection in certain challenge models falls short compared to M protein immunization¹⁴. Moreover, SLO-deficient GAS strains exhibit decreased virulence in mouse models¹⁵, while serum from SLO- or SLO toxoid-immunized mice can protect naive mice against challenges with wild-type strains.

SLO is highly conserved and found in more than 95% of all characterized S. pyogenes isolates across the world, positioning SLO as a notable vaccine candidate. In fact, SLO is included as one of the antigens in at least three multivalent vaccines under investigation in pre-clinical and clinical trials¹⁶. Current SLO-based toxoid vaccines have been developed by introducing point amino acid substitutions primarily in the membrane-binding loop of Domain 4 to eliminate cytolysis effect during immunization^{11,17}. Unlike the M-protein, there have been no reports to date of functional monoclonal antibodies with known primary amino acid sequences against SLO, which impedes the identification of the protective epitope(s). In contrast, several hemolysis-inhibiting mAbs have been characterized for pneumolysin O, a homologous CDC secreted by Streptococcus pneumoniae¹⁸. However, high-resolution epitope mapping using overlapping peptide fragments proved ineffective as the 3-D conformation was lost using this approach.

A recent review describing the *Streptococcus pyogenes* vaccine landscape is Walkinshaw et al (2023)²⁸.

Protein nanoparticles have been used for viral vaccines where antigens are displayed on the surface of the protein nanoparticles, Walls et al 2000²⁷.

There is a need for improved vaccines the treatment and prevention of bacterial infections, such as from *Streptococcus pyogenes.* In particular there is a need for suitable epitopes and immunogenic proteins and complexes comprising these epitopes to protect against and treat diseases from bacterial infections.

### Summary of the invention

The object of the present invention is to overcome certain drawbacks in bacterial vaccines used in the treatment or prevention of bacterial infections, such as from *Streptococcus pyogenes.* This is achieved by the present inventors who have designed novel Streptolysin O epitopes, and immunogenic proteins, nanoparticles and pharmaceutical compositions comprising the same.

In this study, we employed a multi-mass spectrometry approach to model a SLO-neutralizing antibody complex in its native conformation to identify and investigate the protective epitope. Initially, de novo mass spectrometry sequencing was used to determine the primary amino acid sequence of a neutralizing monoclonal antibody (nAb) with previously unknown protein sequence. This was subsequently used to generate antigen-antibody distant constraints by chemical cross-linking mass spectrometry. The present inventors defined the epitope by utilizing an information-driven docking protocol to predict the most important amino acids in the protein binding interface between Fab domain of nAb and SLO. The epitope, located in Domain 3, is conserved across all GAS and Group G streptococcus (GGS) genomes thus giving the potential for novel effective vaccines againt *S. pyogenes.*

The present invention provides in a first aspect a Streptolysin O (SLO) epitope variant comprising the two discontinuous parts of Domain 3 linked together by a linker polypeptide replacing the extended loop part of Domain 1 and causing the SLO epitope variant to fold into substantially the same quaternary structure as the SLO epitope in *Streptococcus pyogenes* serotype M3 (strain SSI-1).

In one embodiment the SLO epitope variant has the structure :

X1 - L1 - X2,

wherein X1 is the first discontinuous part of Domain 3, L1 is the linker polypeptide replacing the extended loop part of Domain 1, and X2 is the second discontinuous part of Domain 3.

In a second aspect the present invention provides an immunogenic protein comprising a SLO epitope variant as defined in the first aspect if the invention.

In a third aspect the present invention provides a fusion protein comprising at least one SLO epitope variant as defined in the first aspect if the invention and at least one nanoparticle trimeric component.

In a fourth aspect the present invention provides a protein nanoparticle comprising a SLO epitope variant as defined in the first aspect of the invention.

In a fifth aspect the present invention provides an antibody binding to a SLO epitope variant as defined in the first aspect of the invention.

In a sixth aspect the present invention provides a nucleic acid molecule encoding a SLO epitope variant, an immunogenic protein, a fusion protein, a protein nanoparticle or an antibody as defined in the above mentioned aspects.

In a seventh aspect the present invention provides an expression vector comprising the nucleic acid molecule as defined in the sixth aspect and a suitable promoter.

In another aspect the present invention provides a host cell comprising the nucleic acid molecule as defined in the sixth aspect or the expression vector as defined in the seventh aspect.

In another aspect the present invention provides a pharmaceutical composition comprising a SLO epitope variant, an immunogenic protein, a fusion protein, a protein nanoparticle or an antibody as defined in any one of the above aspects, and a pharmaceutically acceptable excipient.

The present invention provides and is further characterized by the following items:
1. A Streptolysin O (SLO) epitope variant comprising the two discontinuous parts of Domain 3 linked together by a linker polypeptide replacing the extended loop part of Domain 1 and causing the SLO epitope variant to fold into substantially the same quaternary structure as the SLO epitope in *Streptococcus pyogenes* serotype M3 (strain SSI-1).
2. The SLO epitope variant according to item 1, having the structure :

   X1- L1- X2,

   wherein X1 is the first discontinuous part of Domain 3, L1 is the linker polypeptide replacing the extended loop part of Domain 1, and X2 is the second discontinuous part of Domain 3.
3. The SLO epitope variant according to any one of items 1-2, wherein said linker polypeptide (L1) replacing the extended loop part of Domain 1 has a length of less than 12 amino acid residues.
4. The SLO epitope variant according to any one of items 1-3, wherein said linker polypeptide (L1) replacing the Extended loop part of Domain 1 (L1) is selected from the group consisting of APNG, APGG, CPNG and CPGG.
5. The SLO epitope variant according to any one of items 1-4, wherein said extended loop part of Domain 1 is SEQ ID NO: 4.
6. The SLO epitope variant according to any of items 1-5, wherein said first discontinuous part of Domain 3 (X1) has at least 80%, at least 87%, at least 89%, at least 93% or at least 95% amino acid identity to the polypeptide SEQ ID NO: 3.
7. The SLO epitope variant according to any of items 1-6, wherein said first discontinuous part of Domain 3 (X1) has at least 72%, at least 77%, at least 83%, at least 93% or at least 96% amino acid similarity to the polypeptide SEQ ID NO: 3.
8. The SLO epitope variant according to any of items 1-7, wherein said first discontinuous part of Domain 3 (X1) is the polypeptide SEQ ID NO: 3.
9. The SLO epitope variant according to any one of items 1-8, wherein said second discontinuous part of Domain 3 (X2) has at least 81%, at least 85%, at least 89%, at least 93% or at least 97% amino acid identity to the polypeptide SEQ ID NO: 5.
10. The SLO epitope variant according to any one of items 1-9, wherein said second discontinuous part of Domain 3 (X2) has at least 72%, at least 79%, at least 86%, at least 93% or at least 97% amino acid identity to the polypeptide SEQ ID NO: 5.
11. The SLO epitope variant according to any of items 1-10, wherein said second discontinuous part of Domain 3 (X2) is the polypeptide SEQ ID NO: 5.
12. The SLO epitope variant according to any one of items 1-11, which is selected from the group consisting of SEQ ID NO: 6, SEQ ID NO:7, SEQ ID NO: 8 and SEQ ID NO:9.
13. The SLO epitope variant according to any one of items 1-12, which is SEQ ID NO: 6.
14. The SLO epitope variant according to any one of items 1-12, which is SEQ ID NO: 7.
15. The SLO epitope variant according to any one of items 1-12, which is SEQ ID NO: 8.
16. The SLO epitope variant according to any one of items 1-12, which is SEQ ID NO: 9.
17. An immunogenic protein comprising a SLO epitope variant as defined in any one of items 1-16.
18. A fusion protein comprising at least one SLO epitope variant as defined in any one of items 1-16 and at least one nanoparticle trimeric component.
19. The fusion protein according to item 18, comprising a short linker sequence between said at least one SLO epitope variant or immunogenic protein and said at least one nanoparticle trimeric component.
20. The fusion protein according to item 19, wherein said short linker sequence has from 2 to 4 amino acid residues.
21. The fusion protein according to item 19 or 20, wherein said short linker sequence is SA or GGSA.
22. The fusion protein according to any one of items 18-21, wherein said nano-particle trimeric component is I53_dn5B (SEQ ID NO: 10).
23. The fusion protein according to any one of items 18-20, comprising at the C-terminal of said trimeric component a flexible linker having from 2 to 4 amino acid residues, and a protease recognition sequence.
24. The fusion protein according to item 23, wherein said protease recognition sequence is a WELQut protease recognition sequence, such as WELQ (SEQ ID NO: 11).
25. The fusion protein according to item 23 or 24, further comprising a C-terminal oligo-histidine sequence.
26. The fusion protein according to item 25, wherein said oligo-histidine sequence comprises from 4-10 histidines, such as HHHHHH (SEQ ID NO:12).
27. A protein nanoparticle comprising a SLO epitope variant as defined in any one of items 1-16.
28. The protein nanoparticle according to item 27, comprising at least two of said SLO epitope variants, at least 4 SLO epitope variants or at least 8 SLO epitope variants.
29. The protein nanoparticle according to item 28, wherein said SLO epitope variants are all identical.
30. The protein nanoparticle according to any one of item 29, wherein at least two of said SLO epitope variants are different.
31. The protein nanoparticle according to item 30, wherein said SLO epitope variants are all different.
32. A protein nanoparticle comprising a fusion protein as defined in any one of items 18-26.
33. The protein nanoparticle according to item 32, comprising at least two of said SLO epitope variants, at least 4 SLO epitope variants or at least 8 SLO epitope variants.
34. The protein nanoparticle according to item 33, wherein said SLO epitope variants are all identical.
35. The protein nanoparticle according to any one of item 33, wherein at least two of said SLO epitope variants are different.
36. The protein nanoparticle according to item 35, wherein said SLO epitope variants are all different.
37. The protein nanoparticle according to any one of items 27-36 for the treatment or prophylactics of a *S. pyogenes* associated disease, disorder or condition.
38. An antibody binding to a SLO epitope variant as defined in any one of items 1-16.
39. A nucleic acid molecule encoding a SLO epitope variant, an immunogenic protein, a fusion protein, a protein nanoparticle or an antibody as defined in any one of items 1-38.
40. An expression vector comprising the nucleic acid molecule as defined in item 39 and a suitable promoter.
41. A host cell comprising the nucleic acid molecule as defined in item 39 or the expression vector as defined in item 40.
42. A pharmaceutical composition comprising a SLO epitope variant, an immunogenic protein, a fusion protein, a protein nanoparticle or an antibody as defined in any one of items 1-38, and a pharmaceutically acceptable excipient.
43. The pharmaceutical composition according to item 42, further comprising an adjuvant.
44. The pharmaceutical composition according to item 43, wherein said adjuvant is aluminium hydroxide (alum) or CpG, such as CpG1018.
45. A composition comprising a SLO epitope variant, an immunogenic protein, a fusion protein, a protein nanoparticle or an antibody as defined in any one of items 1-38, for use in the treatment or prophylactics of a S. *pyogenes* associated disease, disorder or condition.
46. A method for the treatment or prophylactics of a S. *pyogenes* associated disease, disorder or condition comprising administering a therapeutically effective amount of a SLO epitope variant, an immunogenic protein, a fusion protein, a protein nanoparticle, an antibody or a pharmaceutical composition as defined in any one of items 1-38 and 42-44.
47. A method for the treatment of a human comprising administration of a therapeutically or prophylactically effective amount of a SLO epitope variant, an immunogenic protein, a fusion protein, a protein nanoparticle, an antibody or a pharmaceutical composition as defined in any one of items 1-38 and 42-44.
48. A method for immunizing a human comprising the administration of an effective amount of a SLO epitope variant, an immunogenic protein, a fusion protein, a protein nanoparticle, an antibody or a pharmaceutical composition as defined in any one of items 1-38 and 42-44.

### Brief description of the figures

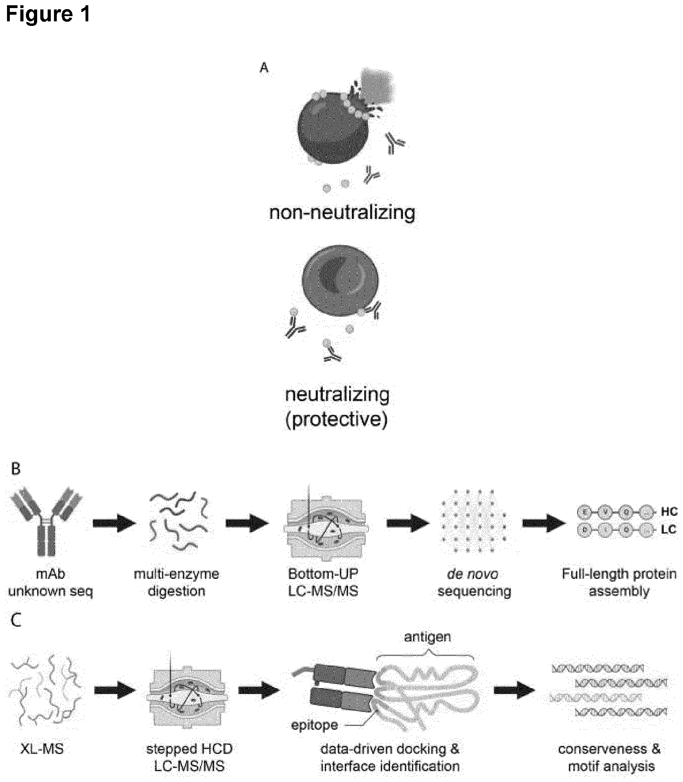

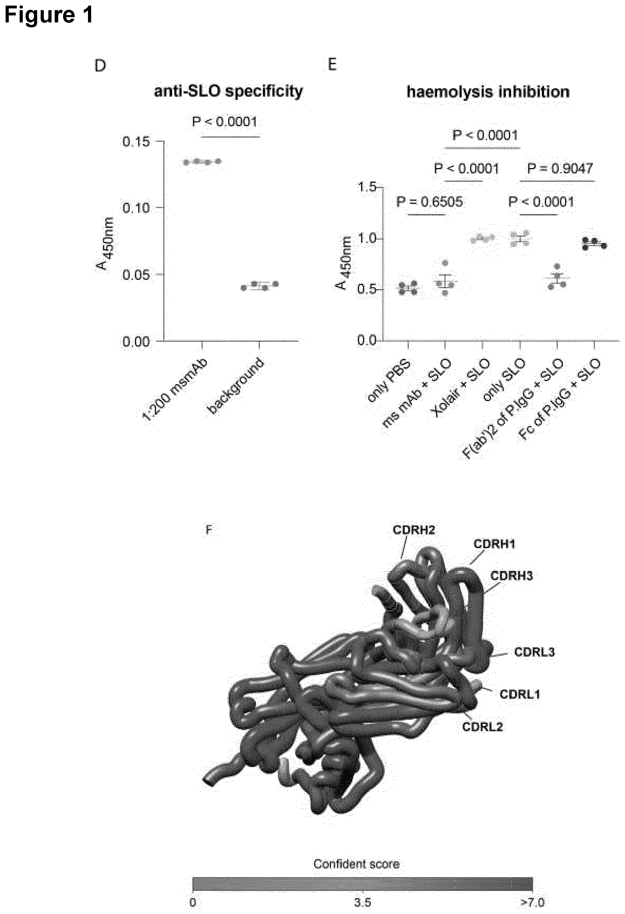

**Fig.1 A)****.** Schematic representation of the inhibition assay used to select neutralizing antibodies (nAb). Graphical illustration of the workflow outlining **B).** the de novo protein sequencing of the nAb, combining multi-enzyme digestion, bottom-up mass spectrometry de novo sequencing, and sequence assembly to obtain complete heavy and light chain information and **C).** cross-linking mass spectrometry (XL-MS) to define XL distance constraints for exploratory modelling, pairwise antigen-antibody complex docking, and assessment of residues involved in the predicted binding interface. **D).** Enzyme-linked immunosorbent assay (ELISA) analysis used to select SLO-specific antibodies. **E).** The neutralization potency was assessed by co-incubating different antibody samples with wild-type SLO, followed by the introduction of diluted sheep red blood cells. The antibody-mediated inhibition of haemolysis was quantified by measuring the amount of released haemoglobin in the supernatant under specific wavelength. P values of two-sample t test and ANOVA are indicated. **F).** A tertiary model of the de novo sequenced Fab domain, with the confidence score for each residue denoted by the colour gradient. **G).** To validate the model, XL-MS was applied to study crosslinked nAb. Inter-protein (red) and intra-protein (blue) crosslinks are displayed as pseudo bonds within an AlphaFold-Multimer-predicted Fab structure. A bar plot, with the y-axis representing XL count and the x-axis representing Cα-Cα distances for all the identified crosslinks. Light and heavy chains are distinguished by colour codes, and the proABC-2-predicted hypervariable (HV) loop regions are highlighted by yellow or orange.

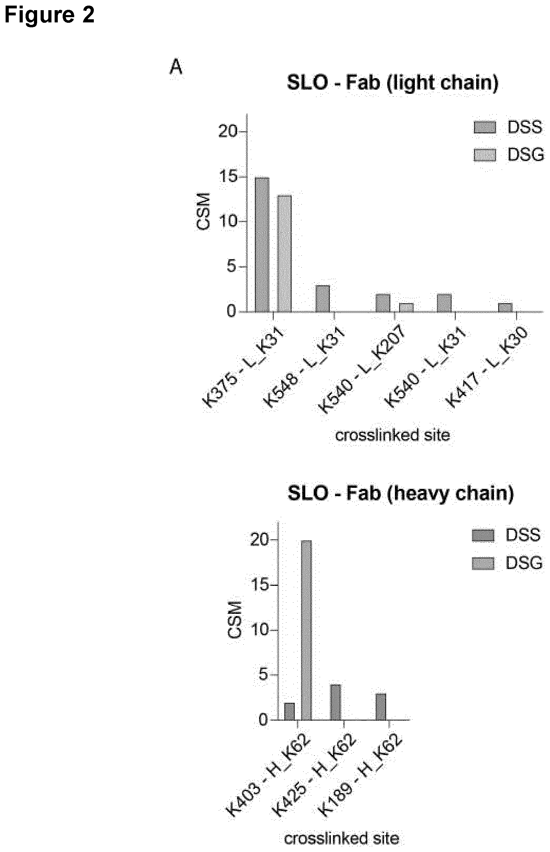

**Fig.2 A)****.** The number of crosslinked spectrum match (CSM) for the identified interprotein DSG- and DSS-crosslinked sites between SLO-Fab light chain and SLO-Fab heavy chain, **B).** Representative MS/MS spectra of the two predominant XL constraints are illustrated with annotated fragment ions. The top panel and lower panel represent the SLO(K376)-Fab_LC(K31) and the SLO(K403)-Fab_HC(K62) crosslinked site, respectively. **C).** DisVis Quick scanning analysis was used to filter experimental distant restraints for Fab-SLO modelling. Each crosslinked site (restraint) is plotted on the x-axis with the z-score value on the y-axis. The encircled group of 4 restraints with low z-scores was used for downstream analysis. **D).** The number of accessible SLO-Fab complexes based on the number of utilized XL distance constraints. The 44'674 complexes remaining after using 4 XL restraints was used for downstream analysis. **E).** The accessible interaction space based on the filtered 44'674 complexes shown as a shell centred around Domain 3 in SLO. **F).** Heat maps displaying the IF score of each residue from the light and the heavy chain respectively, with a colour bar with the IF value ranging from 0 to 3. **G).** Heatmap showing the IF index of all accessible SLO residue across based on the number of distance restraints used. Colour bar shows IF value from 0 to 4.6. **H).** Left, display of the top ranking IF-derived interactive residues with sidechain shown as sticks on the tertiary structure coloured in blue. Right, overview of conformational epitope in the SLO 3D crystal structure colored in purple.

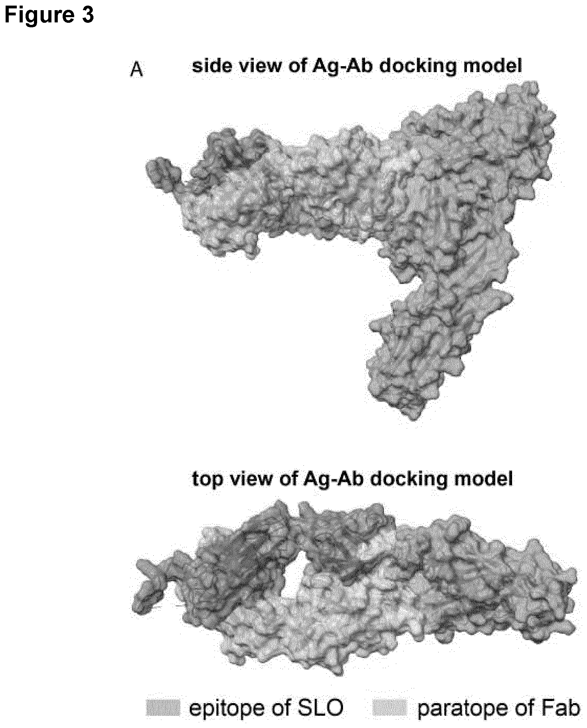

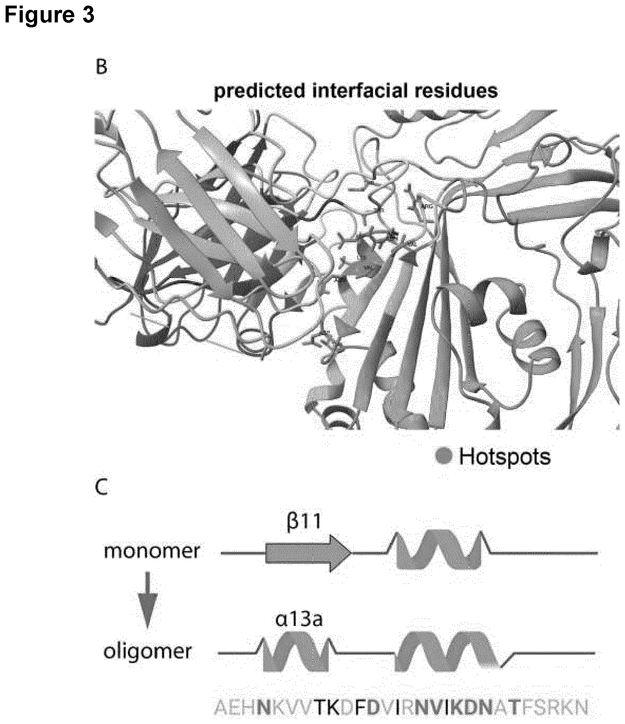

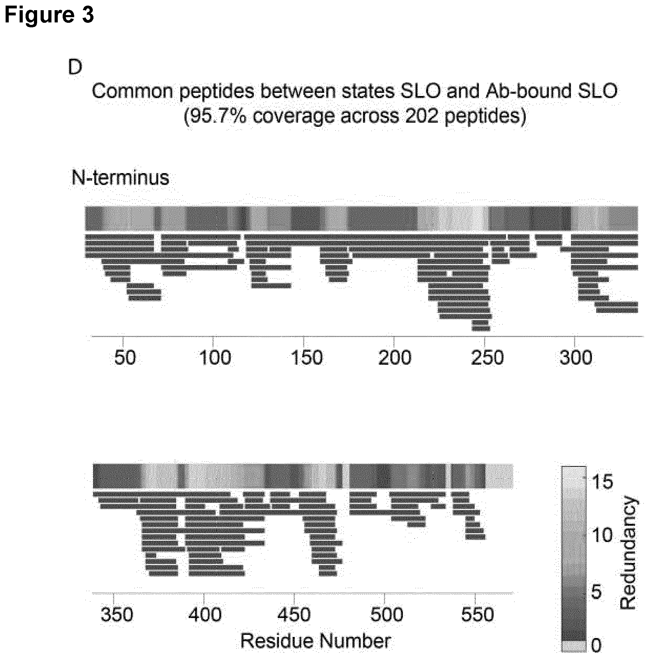

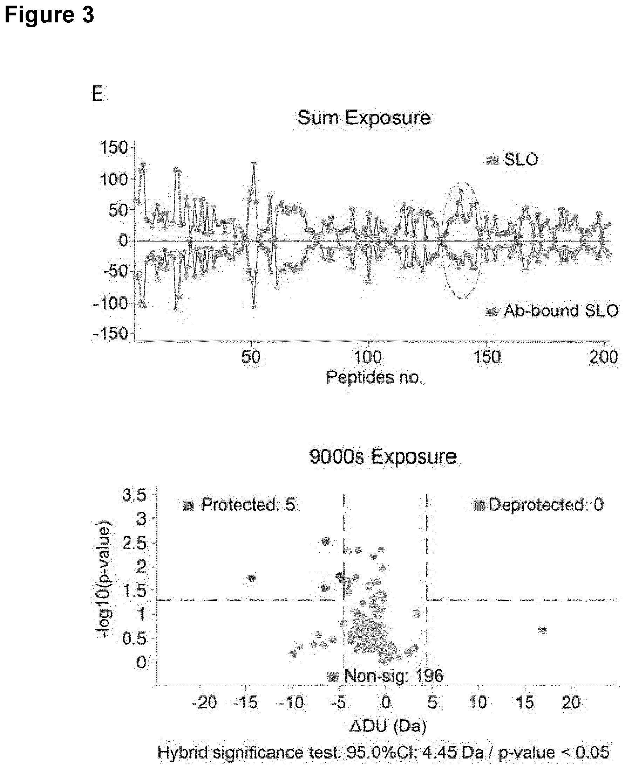

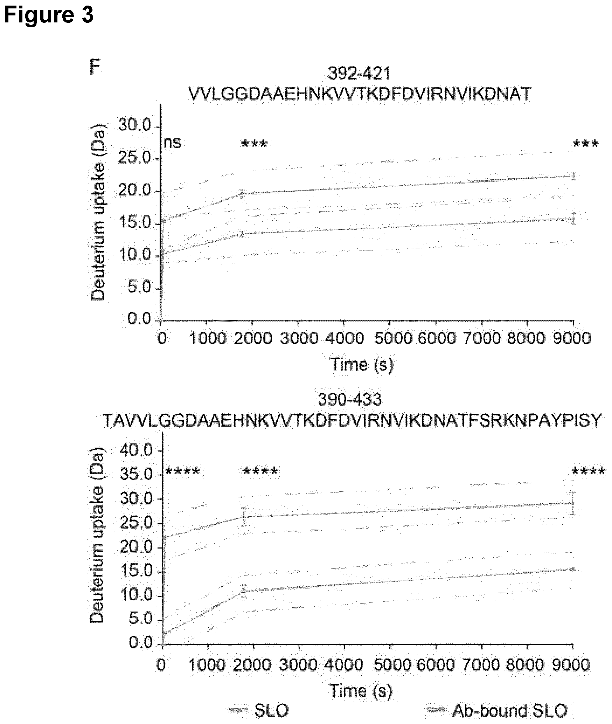

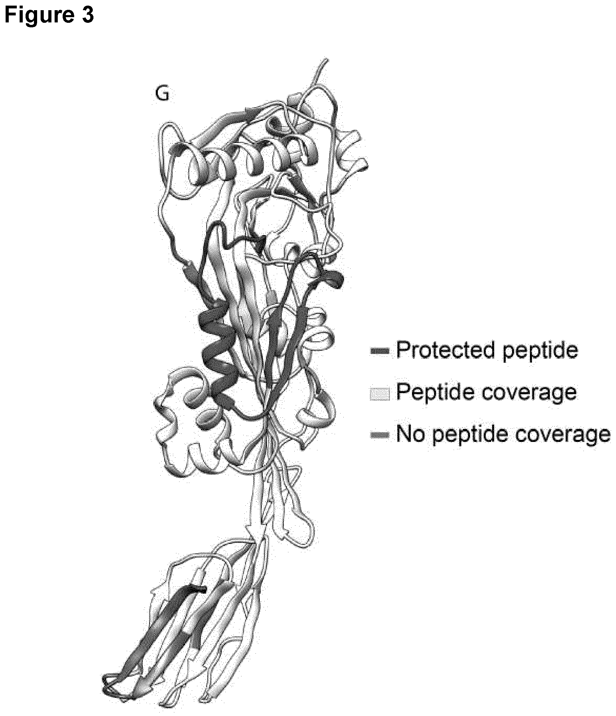

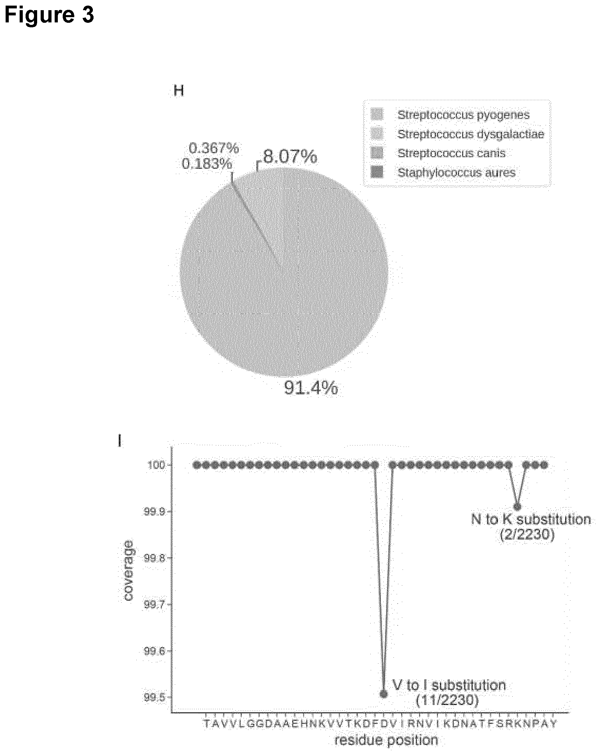

**Figure 3. A).** Top-ranking model generated by HADDOCK 2.4 antigen-antibody docking protocol with a loose epitope definition and the two frequently observed XL restraints as input. The predicted epitope and paratope from the complex are colour-coded correspondingly. **B).** Interface residues were identified and classified from the optimal Fab-SLO model, with hotspots highlighted in hotpink to represent residues predicted to contribute most significantly to the binding. **C).** Streptolysin O, similar to other CDC proteins, undergoes conformational changes in the secondary structure of Domain 3. The Fab-recognized epitope overlaps a portion of these structurally critical motifs. **D).** The percentage of sequence conservation the critical residues for antigen-antibody binding across 2083 sequenced GAS genomes. The redundancy map of 202 common peptides identified in both SLO and Ab-bound SLO conditions from HDX-MS experiments. Peptides are displayed in varying lengths, with residue redundancy visualized by a colour gradient from 0 to 16. **E).** The cumulative Da changes due to deuterium uptake across all peptides are shown in a butterfly plot, which compares SLO and Ab-bound SLO conditions. A volcano plot presents protected and deprotected peptides, with thresholds set at |ΔDU| > 3.79 Da and p-value from hybrid significance test < 0.05. **F).** Kinetic plots display the two most representative protected peptides of SLO under Ab binding at 0s, 60s, 1800s, and 9000s deuteration intervals. Thresholds were globally calculated with 'ns' as non-significant, '***' as ≥99% significance and '****' as ≥99.9% significance. **G).** The 3D model of SLO showcases projected protected peptides following 9000s deuteration. Here, residues from protected peptides are highlighted in blue, while absent residues are depicted in dark grey. **H-I)** Carriage and of SLO across four streptococcal species and the degree of amino acid sequence conservation of the epitope.

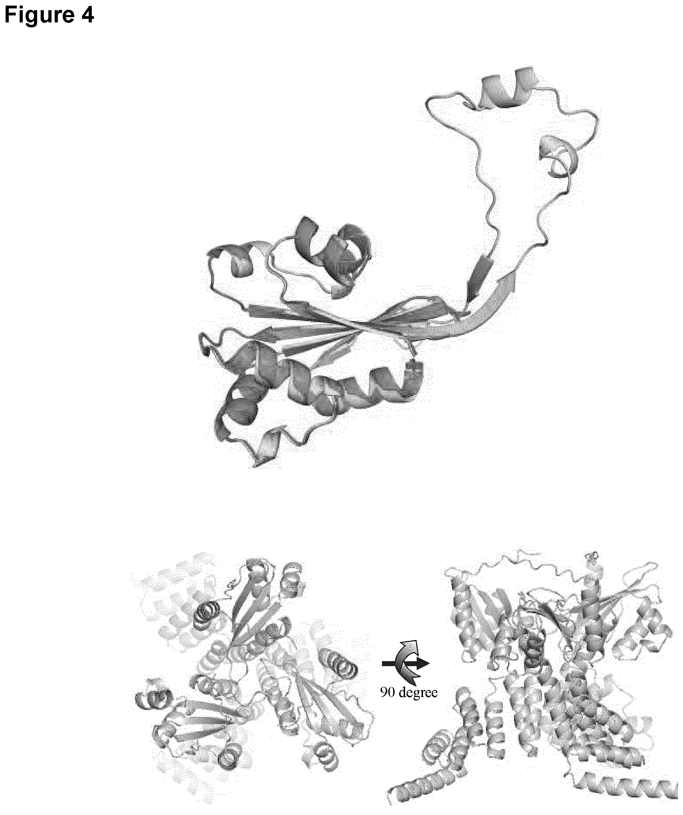

**Figure 4****. a)** ProteinMPNN and Alpha2Fold modelling of the epitope (magenta), compared to the known structure of the protein domain (green). **b)** Modelled epitope (purple) on designed trimers (green).

### Detailed description of the invention

Unless specifically defined herein, all technical and scientific terms used have the same meaning as commonly understood by a skilled artisan in the fields of immunology, biochemistry, genetics, and microbiology.

All methods and materials similar or equivalent to those described herein can be used in the practice or testing of the present invention, with suitable methods and materials being described herein. All publications, patent applications, patents, and other references mentioned herein are incorporated by reference in their entirety. In case of conflict, the present specification, including definitions, will prevail. Further, the materials, methods, and examples are illustrative only and are not intended to be limiting, unless otherwise specified.

The practice of the present invention will employ, unless otherwise indicated, conventional techniques of cell biology, cell culture, molecular biology, transgenic biology, microbiology, and recombinant DNA, which are within the skill of the art. Such techniques are explained fully in the literature. See, for example, Current Protocols in Molecular Biology (Frederick M. AUSUBEL, 2000, Wiley and son Inc, Library of Congress, USA); Molecular Cloning: A Laboratory Manual, Third Edition, (Sambrook et al, 2001, Cold Spring Harbor, New York: Cold Spring Harbor Laboratory Press); Oligonucleotide Synthesis (M. J. Gait ed., 1984); Mullis et al. U.S. Pat. No. 4,683,195; Nucleic Acid Hybridization (B. D. Harries & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the series, Methods In ENZYMOLOGY (J. Abelson and M. Simon, eds.-in-chief, Academic Press, Inc., New York), specifically, Vols.154 and 155 (Wu et al. eds.) and Vol. 185, "Gene Expression Technology" (D. Goeddel, ed.); Gene Transfer Vectors For Mammalian Cells (J. H. Miller and M. P. Calos eds., 1987, Cold Spring Harbor Laboratory). Kuby Immunology (2018, J Punt & S Stranford). Therapeutic Antibody Engineering (2012, 1st Ed), W Strohl & L Strohl.

The present invention provides a Streptolysin O (SLO) epitope variant comprising the two discontinuous parts of Domain 3 linked together by a linker polypeptide replacing the extended loop part of Domain 1 and causing the SLO epitope variant to fold into substantially the same quaternary structure as the SLO epitope in *Streptococcus pyogenes* serotype M3 (strain SSI-1).

In one embodiment the SLO epitope variant has the structure :

X1 - L1 - X2,

wherein X1 is the first discontinuous part of Domain 3, L1 is the linker polypeptide replacing the extended loop part of Domain 1, and X2 is the second discontinuous part of Domain 3.

In another embodiment the linker polypeptide (L1) replacing the extended loop part of Domain 1 has a length of less than 12 amino acid residues. In another embodiment the linker polypeptide (L1) replacing the Extended loop part of Domain 1 (L1) is selected from the group consisting of APNG, APGG, CPNG and CPGG.

In another embodiment the extended loop part of Domain 1 is SEQ ID NO: 4.

In another embodiment the first discontinuous part of Domain 3 (X1) has at least 80%, at least 87%, at least 89%, at least 93% or at least 95% amino acid identity to the polypeptide SEQ ID NO: 3.

In another embodiment the first discontinuous part of Domain 3 (X1) has at least 72%, at least 77%, at least 83%, at least 93% or at least 96% amino acid similarity to the polypeptide SEQ ID NO: 3. In yet another embodiment the first discontinuous part of Domain 3 (X1) is the polypeptide SEQ ID NO: 3.

In another embodiment the said second discontinuous part of Domain 3 (X2) has at least 81%, at least 85%, at least 89%, at least 93% or at least 97% amino acid identity to the polypeptide SEQ ID NO: 5.

In another embodiment the second discontinuous part of Domain 3 (X2) has at least 72%, at least 79%, at least 86%, at least 93% or at least 97% amino acid identity to the polypeptide SEQ ID NO: 5. In yet another embodiment the second discontinuous part of Domain 3 (X2) is the polypeptide SEQ ID NO: 5.

In another embodiment the SLO epitope variant is selected from the group consisting of SEQ ID NO: 6, SEQ ID NO:7, SEQ ID NO: 8 and SEQ ID NO:9. In yet another embodiment the SLO epitope variant is SEQ ID NO: 6. In another embodiment the SLO epitope variant is SEQ ID NO: 7. In another embodiment the SLO epitope variant is SEQ ID NO: 8. In another embodiment the SLO epitope variant is SEQ ID NO: 9.

In an aspect the present invention provides an immunogenic protein comprising a SLO epitope variant as defined above.

In another aspect the present invention provides a fusion protein comprising at least one SLO epitope variant as defined above and at least one nanoparticle trimeric component.

In one embodiment the fusion protein comprises a short linker sequence between said at least one SLO epitope variant or immunogenic protein and said at least one nanoparticle trimeric component. In an embodiment the short linker sequence has from 2 to 4 amino acid residues. In another embodiment the short linker sequence is SA or GGSA.

In another embodiment the nano-particle trimeric component is I53_dn5B (SEQ ID NO: 10).

In another embodiment the fusion protein comprises at the C-terminal of said trimeric component a flexible linker having from 2 to 4 amino acid residues, and a protease recognition sequence. In another embodiment this protease recognition sequence is a WELQut protease recognition sequence, such as WELQ (SEQ ID NO: 11).

In another embodiment the fusion protein further comprises a C-terminal oligo-histidine sequence. In another embodiment this oligo-histidine sequence comprises from 4-10 histidines, such as HHHHHH (SEQ ID NO:12).

In another aspect the present invention provides a protein nanoparticle comprising a SLO epitope variant as defined above.

In an embodiment the protein nanoparticle comprises at least two of said SLO epitope variants, at least 4 SLO epitope variants or at least 8 SLO epitope variants.

In another embodiment the SLO epitope variants are all identical.

In another embodiment the protein nanoparticle has at least two of said SLO epitope variants being different.

In another embodiment the protein nanoparticle has all of the SLO epitope variants being different.

In another aspect the present invention provides a protein nanoparticle comprising a fusion protein as defined above.

In another embodiment the protein nanoparticle comprises at least two SLO epitope variants, at least 4 SLO epitope variants or at least 8 SLO epitope variants.

In another embodiment the protein nanoparticle has all the SLO epitope variants being identical.

In another embodiment the protein nanoparticle has at least two of said SLO epitope variants being different.

In another embodiment the protein nanoparticle has all of said SLO epitope variants being different.

In a further aspect the present invention provides the protein nanoparticle according to the invention for the treatment or prophylactics of a S. *pyogenes* associated disease, disorder or condition.

In a further aspect the present invention provides an antibody binding to a SLO epitope variant as defined above.

In yet a further aspect the present invention provides a nucleic acid molecule encoding a SLO epitope variant, an immunogenic protein, a fusion protein, a protein nanoparticle or an antibody as defined above.

In a further aspect the present invention provides an expression vector comprising the nucleic acid molecule as defined above and a suitable promoter.

In a further aspect the present invention provides a host cell comprising the nucleic acid molecule as defined above or the expression vector as defined above.

In a further aspect the present invention provides a pharmaceutical composition comprising a SLO epitope variant, an immunogenic protein, a fusion protein, a protein nanoparticle or an antibody as defined above, and a pharmaceutically acceptable excipient.

In one embodiment the pharmaceutical composition further comprises an adjuvant.

In another embodiment the pharmaceutical composition comprises an adjuvant which is aluminium hydroxide (alum) or CpG, such as CpG1018.

In a further aspect the present invention provides a composition comprising a SLO epitope variant, an immunogenic protein, a fusion protein, a protein nanoparticle or an antibody as defined above, for use in the treatment or prophylactics of a S. *pyogenes* associated disease, disorder or condition.

Compositions and formulations in accordance with the present invention are prepared in accordance with known standards for the preparation of pharmaceutical compositions and formulations. For instance, the compositions and formulations are prepared in a way that they can be stored and administered appropriately, e.g. by using pharmaceutically acceptable components such as carriers, excipients or stabilizers. Such pharmaceutically acceptable components are not toxic in the amounts used when administering the pharmaceutical composition or formulation to a patient. The pharmaceutical acceptable components added to the pharmaceutical compositions or formulations may depend on the chemical nature of the inhibitor and targeting agent present in the composition or formulation (depend on whether the targeting agent is e.g. an antibody or fragment thereof or a cell expressing a chimeric antigen receptor), the particular intended use of the pharmaceutical compositions and the route of administration.

In a preferred embodiment in accordance with the invention, the composition or formulation is suitable for administration to humans, preferably the formulation is sterile and/or non-pyrogenic.

### Methods of treatment

In an aspect the present invention provides a method for the treatment or prophylactics of a S. *pyogenes* associated disease, disorder or condition comprising administering a therapeutically effective amount of a SLO epitope variant, an immunogenic protein, a fusion protein, a protein nanoparticle, an antibody or a pharmaceutical composition as defined in the present invention.

In a further aspect the present invention provides a method for the treatment of a human comprising administration of a therapeutically or prophylactically effective amount of a SLO epitope variant, an immunogenic protein, a fusion protein, a protein nanoparticle, an antibody or a pharmaceutical composition as defined by the present invention.

In a further aspect the present invention provides a method for immunizing a human comprising the administration of an effective amount of a SLO epitope variant, an immunogenic protein, a fusion protein, a protein nanoparticle, an antibody or a pharmaceutical composition as defined by the present invention.

### Definition of some terms.

Unless otherwise defined below, the terms used in the present invention shall be understood in accordance with the common meaning known to the person skilled in the art.

The term "antibody" (e.g. a monoclonal antibody) as described herein shall be understood in accordance with the common meaning known to the person skilled in the art. The antibody may be a derivatized antibody or be linked to a different molecule. For example, molecules that may be linked to the antibody are other proteins (e.g. other antibodies), a molecular label (e.g. a fluorescent, luminescent, colored or radioactive molecule), a pharmaceutical and/or a toxic agent. The antibody or antigen-binding portion may be linked directly (e.g. in form of a fusion between two proteins), or via a linker molecule (e.g. any suitable type of chemical linker known in the art).

The term "isolated antibody" as described herein is to be understood as an antibody which is recovered or purified to some extent. In an embodiment that extent means it is free from a multitude of antibodies having a different structure such as more than 25 antibodies..

"Conservative substitution" as described herein shall be understood as a substitution of an amino acid residue with a different residue having a similar side chain, and thus typically involves substitution of the amino acid in the polypeptide with amino acids within the same or similar class of amino acids. By way of example and not limitation, an amino acid with an aliphatic side chain may be substituted with another aliphatic amino acid, e.g., alanine, valine, leucine, and isoleucine; an amino acid with hydroxyl side chain is substituted with another amino acid with a hydroxyl side chain, e.g., serine and threonine; an amino acid having an aromatic side chain is substituted with another amino acid having an aromatic side chain, e.g., phenylalanine, tyrosine, tryptophan, and histidine; an amino acid with a basic side chain is substituted with another amino acid with a basic side chain, e.g., lysine and arginine; an amino acid with an acidic side chain is substituted with another amino acid with an acidic side chain, e.g., aspartic acid or glutamic acid; and a hydrophobic or hydrophilic amino acid is replaced with another hydrophobic or hydrophilic amino acid, respectively.

"Non-conservative substitution" as described herein- shall be understood as a substitution of an amino acid in a polypeptide with an amino acid with significantly differing side chain properties. Non-conservative substitutions may use amino acids between, rather than within, the defined groups and affects (a) the structure of the peptide backbone in the area of the substitution (e.g., proline for glycine) (b) the charge or hydrophobicity, or (c) the bulk of the side chain. By way of example and not limitation, an exemplary non-conservative substitution can be an acidic amino acid substituted with a basic or aliphatic amino acid; an aromatic amino acid substituted with a small amino acid; and a hydrophilic amino acid substituted with a hydrophobic amino acid.

The term "percentage identity" as used herein shall be understood to mean the degree of identity between the amino acid residues of two aligned polypeptide sequences.

The term "percentage similarity" as used herein shall be understood to mean the degree to which the amino acid residues of two aligned polypeptides are similar by way of the residues being identical or conservative substitutions.

The term "bind" or "binding" in relation to antibodies as used herein refers to the forming of a complex with a molecule that is to be bound, e.g. a SLO epitope variant. Binding typically occurs non-covalently by intermolecular forces, such as ionic bonds, hydrogen bonds and Van der Waals forces and is typically reversible. Various methods and assays to determine binding capability are known in the art. Binding is usually a binding with high affinity, wherein the affinity as measured in KD values is preferably less than 1 mM, more preferably less than 100 nM, even more preferably less than 10 nM, even more preferably less than 1 nM, even more preferably less than 100 pM, even more preferably less than 10 pM, and even more preferably less than 1 pM.

As used herein, each occurrence of terms such as "comprising" or "comprises" may optionally be substituted with "consisting of" or "consists of'.

Terms such as "treating", "preventing" or "alleviating" according to the present invention refer to a therapeutic treatment. An assessment of whether or not a therapeutic treatment works can, for instance, be made by assessing whether the treatment prevents or inhibits bacterial infection in the treated patient or patients. Preferably, the prevention or inhibition is statistically significant as assessed by appropriate statistical tests which are known in the art. Inhibition or prevention of infection may be assessed by comparing the degree of bacterial infection in a group of subjects infected or exposed to the bacteria which are administered a nanoparticle vaccine according to the present invention to a control group of subjects that are infected or exposed but who is administered placebo. Alternatively, it may be assessed by comparing a group of infected patients that receive a standard antibacterial treatment of the art plus a treatment according to the invention with a control group of patients that only receive a standard antibacterial treatment of the art. Such studies for assessing the inhibition or prevention of bacterial infection are designed in accordance with accepted standards for clinical studies, e.g. double-blinded, randomized studies with sufficient statistical power.

As used herein, the term "pharmaceutically acceptable" means being approved by a regulatory agency of the Federal or a state government or listed in the U.S. Pharmacopia, European Pharmacopia or other generally recognized pharmacopia for use in mammals, and more particularly in humans. Pharmaceutically acceptable carriers include, but are not limited to, saline, buffered saline, dextrose, water, glycerol, sterile isotonic aqueous buffer, and combinations thereof.

**Table 1. Listing of the sequences referred to in the present description.**

| SEQ ID NO: | Sequence name / ID | Sequence |
|---|---|---|
| 1 | Streptolysin O | MSNKKTFKKYSRVAGLLTAALIIGNLVTANAESNKQNTASTETTTTNEQP KPESSELTTEKAGQKTDDMLNSNDMIKLAPKEMPLESAEKEEKKSEDKK KSEEDHTEEINDKIYSLNYNELEVLAKNGETIENFVPKEGVKKADKFIVIER KKKNINTTPVDISIIDSVTDRTYPAALQLANKGFTENKPDAVVTKRNPQKI HIDLPGMGDKATVEVNDPTYANVSTAIDNLVNQWHDNYSGGNTLPAR TQYTESMVYSKSQIEAALNVNSKILDGTLGIDFKSISKGEKKVMIAAYKQIF YTVSANLPNNPADVFDKSVTFKELQRKGVSNEAPPLFVSNVAYGRTVFV KLETSSKSNDVEAAFSAALKGTDVKTNGKYSDILENSSFTAVVLGGDAAE HNKVVTKDFDVIRNVIKDNATFSRKNPAYPISYTSVFLKNNKIAGVNNRT EYVETTSTEYTSGKINLSHQGAYVAQYEILWDEINYDDKGKEVITKRRWD NNWYSKTSPFSTVIPLGANSRNIRIMARECTGLAWEWWRKVIDERDVK LSKEINVNISGSTLSPYGSITYK TQYTESMVYSKSQIEAALNVNSKILDGTLGIDFKSISKGEKKVMIAAYKQIF YTVSANLPNNPADVFDKSVTFKELQRKGVSNEAPPLFVSNVAYGRTVFV |
| 2 | SLO epitope | TQYTESMVYSKSQIEAALNVNSKILDGTLGIDFKSISKGEKKVMIAAYKQIF YTVSANLPNNPADVFDKSVTFKELQRKGVSNEAPPLFVSNVAYGRTVFV |
| | | KLETSSKSNDVEAAFSAALKGTDVKTNGKYSDILENSSFTAVVLGGDAAE HNKVVTKDFDVIRNVIKDNA |
| 3 | First discount. part of Domain 3 | TQYTESMVYSKSQIEAALNVNSKILDGTLGIDFKSISKGEKKVMIAAYK |
| 4 | Extended loop part of D1 | QIFYTVSANLPNNPADVFDKSVTFKELQRKGVSNEAPPLFVSNVAYG |
| 5 | Second discount. part of Domain 3 | RTVFVKLETSSKSNDVEAAFSAALKGTDVKTNGKYSDILENSSFTAVV LGGDAAEHNKVVTKDFDVIRNVIKDNA |
| 6 | D3_APNG | TQYTESMVYSKSQIEAALNVNSKILDGTLGIDFKSISKGEKKVMIAAYK APNG RTVFVKLETSSKSNDVEAAFSAALKGTDVKTNGKYSDILENSSFTAVV LGGDAAEHNKVVTKDFDVIRNVIKDNA |
| 7 | D3_APGG | TQYTESMVYSKSQIEAALNVNSKILDGTLGIDFKSISKGEKKVMIAAYK APGG RTVFVKLETSSKSNDVEAAFSAALKGTDVKTNGKYSDILENSSFTAVV LGGDAAEHNKVVTKDFDVIRNVIKDNA |
| 8 | D3_CPNG | TQYTESMVYSKSQIEAALNVNSKILDGTLGIDFKSISKGEKKVMIAAYK CPNG RTVFVKLETSSKSNDVEAAFSAALKGTDVKTNGKYSDILENSSFTAVV LGGDAAEHNKVVTKDFDVIRNVIKDNA |
| 9 | D3_CPGG | TQYTESMVYSKSQIEAALNVNSKILDGTLGIDFKSISKGEKKVMIAAYK CPGG RTVFVKLETSSKSNDVEAAFSAALKGTDVKTNGKYSDILENSSFTAVV LGGDAAEHNKVVTKDFDVIRNVIKDNA |
| 10 | I53_dn5B | EEAELAYLLGELAYK LGEYRIAIRAYRIALKRDPNNAEAWYNLGNAYYKQGRYREAIEYYQKALE LDPNNAEAWYNLGNAYYERGEYEEAI EYYRKALRLDPNNADAMQNLLNAKMREE |
| 11 | WELQ | WELQHHHHHH |
| 12 | Oligo-His | HHHHHH |

### Examples

### Example 1. Characterization of mAb properties, de novo sequencing, and Fab structure modeling.

Antibody protection can be divided into neutralization and/or opsonization. In this study, we defined antibody protection by the degree of neutralization of SLO mediated cytolysis caused by monoclonal antibodies (mAbs) (Fig.1A). Neutralizing monoclonal antibodies (nAbs) with unknown primary amino acid sequence were subsequently subjected to MS-based antibody characterization and antibody-antigen interaction analysis, by combining de novo mass spectrometry sequencing, XL-MS, integrated computational modeling and HDX-MS. (Fig.1B-C).

As a starting point, we screened readily available anti-SLO mAbs from public and commercial sources that could bind to SLO with high specificity leading to neutralization of SLO-mediated cytolysis. Using a sandwich ELISA, we identified one mAb that bound specifically to SLO (Fig.1D). Neutralization was assessed by incubating different antibody samples with SLO, followed by the addition of diluted sheep red blood cells. Inhibition of haemolysis was quantified by measuring the reduction of released hemoglobin in the supernatant. At a 1:1 molar ration, the mAb mediated complete neutralization of SLO-mediated haemolysis compared to the incubation with SLO alone (Fig.1E). Concurrently, we also demonstrated that the protective effect was mediated by the Fab domain only, as evidenced by comparison with F(ab')2 and Fc fragments derived from convalescent plasma IgG isolates.

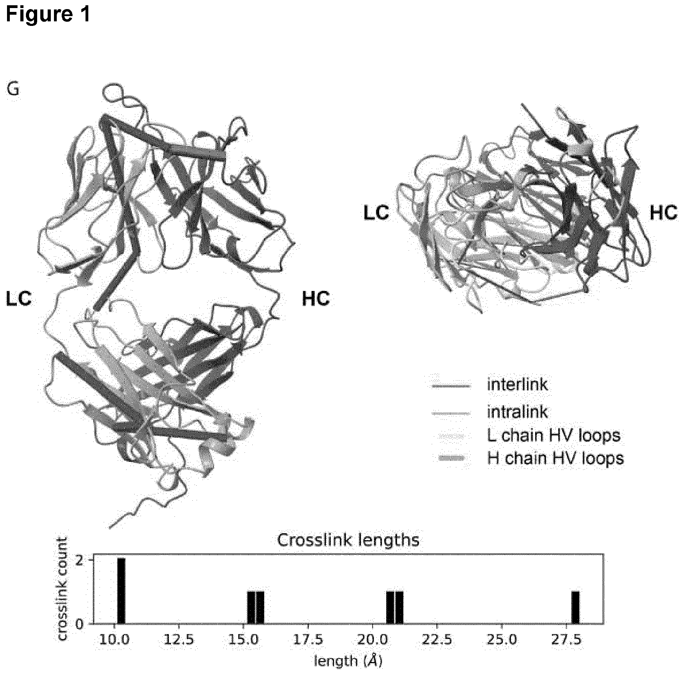

To generate the full amino acid sequence of the nAb, we digested the antibody using different proteases with different cleavage specificity and used a combined de novo sequencing strategy based on three different search engines, resulting in sequences of overlapping proteolytically derived peptide fragments (Gueto-Tettay, C., et al., unpublished work). The overlapping peptide fragments were assembled into a high-confidence full-length sequence for both the light and heavy chains followed by modelling of tertiary structure of the Fab region. The de novo sequencing score of each residue indicates a high sequence confidence for all amino acid residues across the antibody framework, including the complementary determining regions (CDRs) involved in antigen binding (Fig. H). To validate the model, we used AlphaFold-Multimer to model the Fab structure followed by XL-MS to generate intra- and inter- molecular distant constraints within and between the heavy and the light chain. All identified interlinks and intralinks conformed to the modelled Fab structure without violating the Cα-Cα upper distance limit of 30 Å. Furthermore, prediction of the antibody residues and type of interactions involved in the intermolecular interactions was determined by proABC-2 (Fig.1G). Collectively, the identified distant constraints shows that the primary amino acid sequence and the structural Fab model is of sufficient quality to search for the protective epitope in SLO.

### Example 2. Exploratory modeling of interaction based on XL-MS distant constraints

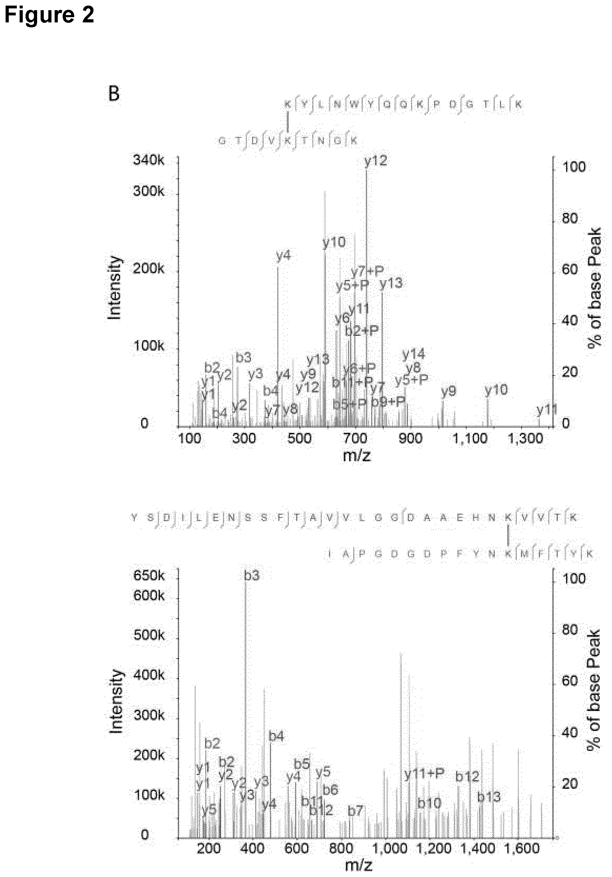

To identify the epitope targeted by the nAb, we incubated SLO at a 1:1: molar ratio with nAb followed by crosslinking using DSG or DSS, identifying in total 8 significant cross-linked peptide pairs (5 for SLO-Fab_LC and 3 SLO-Fab_HC). The DGS and DSS cross-linker reagents have different lengths of the spacer arm resulting in partially overlapping cross-linked peptide pairs (Fig. 2A). Three of the cross-linked peptide pairs were identified by several independent crosslinked spectra matches (CSM) for both DSS and DSG (Fig 2A) and two representative MS/MS spectra are shown for the two most frequently observed crosslinked (XL) peptide pairs in Figure 2B.

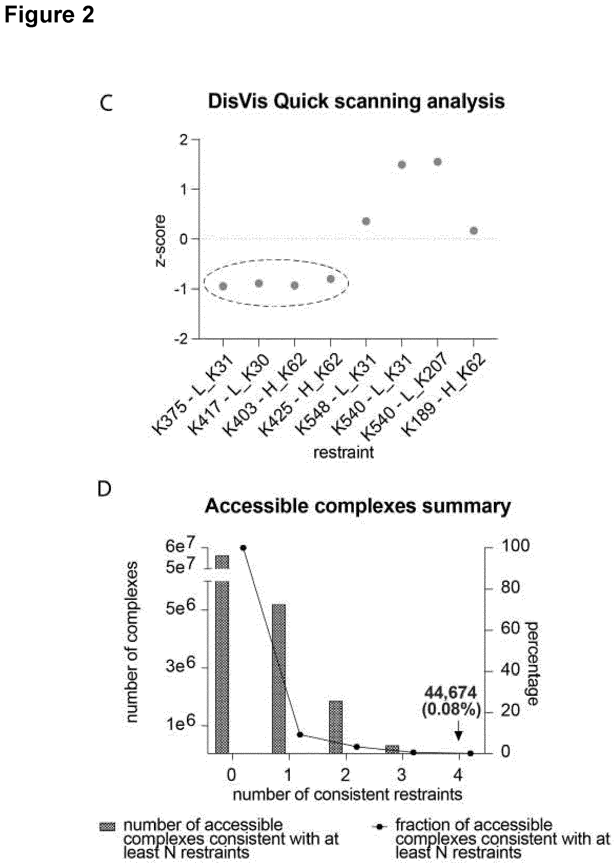

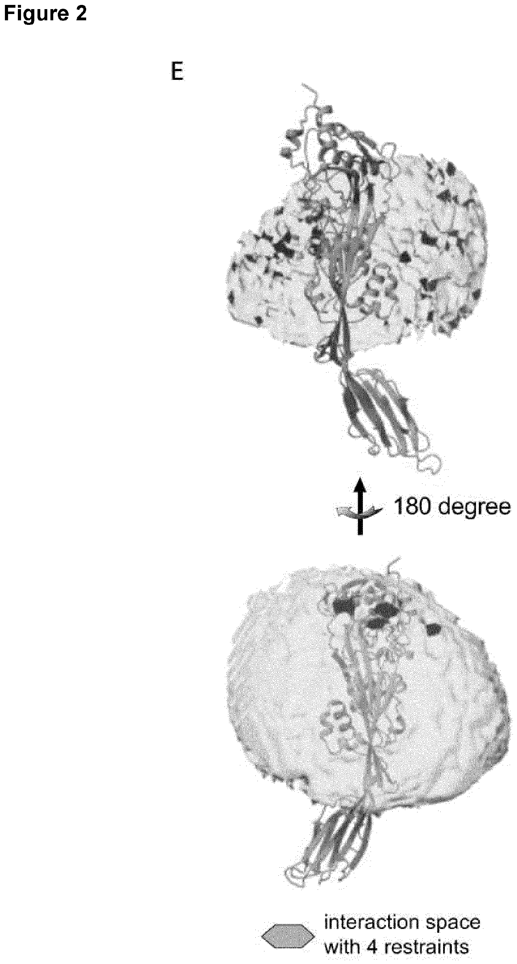

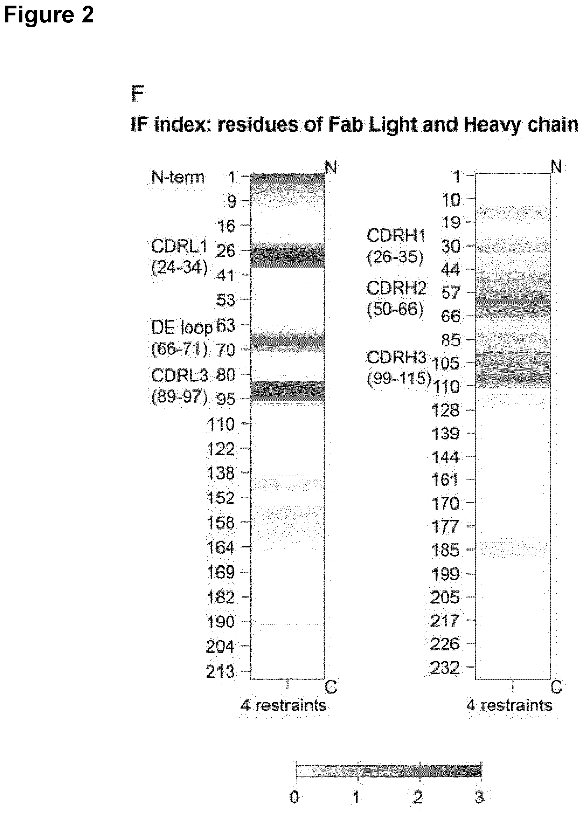

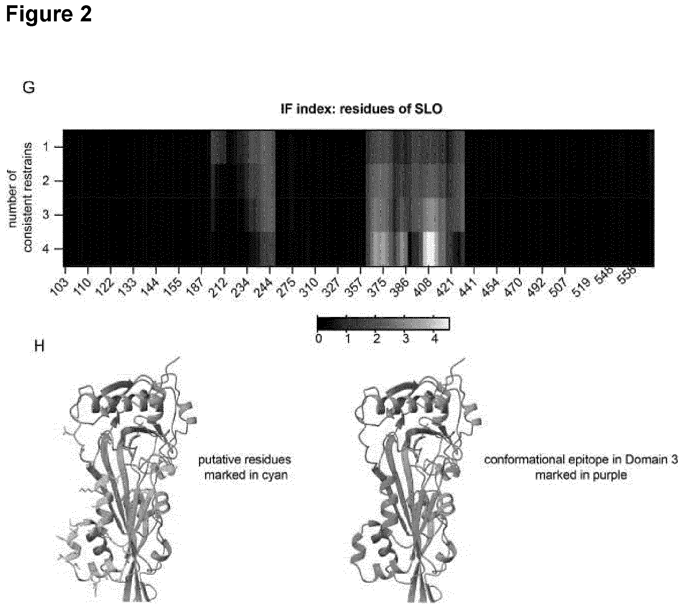

To select the most informative XL peptide pairs, DisVis quick scanning was applied to cluster the identified XLs (Fig.2C). Four XL pairs displayed a negative z-score and was selected for the modelling (Fig.2C), among which the two XL pairs with the highest number of CSMs were included. In the next step, the four filtered XL distance constraints were used to guide the complete DisVis analysis to determine occupancy of residues and the putative binding interface. In the absence of cross-link constraints and with SLO as the fixed chain and Fab as the scanning chain, nearly six million interaction conformations were generated as a starting point. This vast conformational space was produced by allowing the Fab chain to freely translate and rotate relative to the fixed SLO chain. All surface-exposed residues on both interacting partners were considered potential interaction candidates if they had a relevant solvent accessible (RSA) value greater than 0.4. The approach was aimed to provide a complete representation of all possible interaction scenarios between SLO and the Fab fragment. The stepwise implementation of the four most informative cross-links reduced the conformational space by removing conformations that violated the distance constraints, culminating in a final set of 44,674 conformations (0.08%) when including all the four cross-links (Fig.2D). The final interaction space was derived from all possible SLO-Fab complexes using the filtered 44'674 conformations demonstrating that the Fab-targeting epitope resides within the Domain 3 of SLO (Fig.2E). Finally, we used interaction fraction (IF) index to predict the residues of importance for the antigen-antibody binding. On the Fab side, the most important residues with an IF index higher than 0.5 was, as expected, found in the CDRs for both the heavy and the light chain (Fig.2F). The most important residues in SLO were located in two distinct discontinuous regions located between amino acids 187-244 and 357-440 with the latter one being the most important one (Fig.2G). Upon highlighting these regions onto the tertiary crystal structure of SLO shows that the epitope is located within Domain 3 and is composed of helices and sheets (Fig.2H).

### Example 3. Information-driven docking of Fab-SLO complex, conserveness analysis, and epitope mapping

The DisVis analysis, the IF-index along with acquired XL distance constraints enabled us to predict a loosely defined epitope located in Domain 3 of SLO. To further refine the interaction site, we used the HADDOCK 2.4 antigen-antibody docking protocol. This protocol used the interface information from DisVis to generate more accurate starting conformations for iterative docking and refinement. We tested different information inputs such as no epitope information, loose epitope definition, loose epitope definition + XL distance constraints, to guide the docking process and found that the DisVis-derived loose epitope combined with the two most frequently observed XL restraints as the center of mass resulted in the most informative cluster (comprising 240 out of 400 models) . The top-ranked model from this cluster resulted in a high confident interaction site with a combined HADDOCK score of -145 (Fig.3A). Next, the SpotOn algorithm, which predicts possible sidechain interaction between pairs of residues, was applied to identify the most likely residues involved in the formation of the interface, referred to as Hotspots (Fig. 3B). Like other CDC family members, streptolysin O forms a pore on membrane through a multi-stage process that includes initial binding to the cellular membrane followed by oligomerization. During this process Domain 3 undergoes a drastic conformational change, triggering oligomerization, pre-pore formation and final transition to pore. The nAb was found to bind precisely to the critical region responsible for oligomerization preventing the secondary structure changes required for pore-formation (Fig. 3C).

To provide orthogonal evidence for the epitope, we used HDX-MS to investigate how deuterium uptake of SLO residues changes when binding to the nAb. An initial map of peptide coverage was constructed, with the redundancy of the residues represented by a color gradient palette to monitor SLO peptide coverage and to minimize false negatives (Fig.3D). The aggregate exposure of changes in deuterium update (ΔDU) for each of the identified peptides was calculated over four deuteration intervals (0s, 60s, 1800s, and 9000s) and shown in a butterfly plot. Overall, the deuterium uptake of apoSLO and the nAb-SLO complex were similar, except for peptides appearing before the 150th peptide. The peptides spanning the amino residues 392-421, 398-409, 392-422, 391-408, 390-433, 392-409, 390-408, 390-409, 390-410 and 390-422 were significantly protected (Fig.3E). Kinetic plots were created to illustrate deuterium uptake from two representative peptides across the four deuteration intervals, with the level of significance indicating protection by antibody (Fig.3F). Highlighting all protected peptides on the SLO structure revealed the experimentally determined SLO epitope targeted by the nAb (Fig.3G). The result from HDX-MS and XL-MS overlapped supporting the high confident definition of the protective epitope. To determine the which streptococcal species that can produce SLO, we search for SLO orthologues in all streptococcal species. Four species have the SLO gene and 91.4% of searched entries were found in *Streptococcus pyogenes,* indicating a high prevalence of carriage (Fig. 3H). Finally, we assessed the level of conservation for epitope sequence in 2230 publicly available GAS genomes. In total, 99,4% of all genomes carried SLO with a 100% sequence conservation, with only 0.6% of the SLO sequences having a semi-conserved point substitution from valine to isoleucine. (Fig. 3I). These results demonstrates that the neutralizing antibody targets a highly conserved epitope in Domain 3 of SLO to inhibit SLO-mediated cytolysis by preventing changes in the SLO secondary structure and subsequent SLO oligomerization and pore formation.

### Example 4: Design of SLO based self-assembling nanoparticle vaccines

The most important residues in Streptolysin O (SLO) were located in two distinct discontinuous regions located in Domain 3 between amino acids 187-244 and 357-440 with the latter being the most important one. Domain 3 is located between residues 250-299 and 346-420, separated by a subsequence of Domain 1. Due to the discontinuity of the domain 3, the sequence was redesigned for use as a starting point for a vaccine. To this end we used ProteinMPNN to determine which amino acids could replace Domain 1, while the protein backbone of Domain 3 remained fixed. The algorithm generated 32 protein sequence designs, out of which the one with the highest score was chosen. To validate the design, the sequence was modelled with alphafold2 and aligned to the native structure in PyMol (Fig 4). To evaluate the stability of the designed structure, we evaluated whether higher structural stability could be achieved by exchanging any of the internal amino acids. While keeping all surface residues fixed, ProteinMPNN sought to find optional amino acids for the internal residues. The result showed that none of the internal residues generated better fits than the native ones.

In the final step, a nano particle trimeric component was fused to the C-terminal of the designed sequence. The designed D3 domain preceded a short linker sequence (SA and GGSA), followed by the nano particle trimeric component I53_dn5B with a C-terminal flexible linker (GG), a WELQut protease recognition sequence (WELQ) and a hexa-histidine tag. To validate that the structure of the designed domain remained fixed, the vector was modelled using alphafold2 and the designed domain was aligned to the native structure in PyMol (Fig. 4). A multimer, consisting of three vector components were folded and assembled in Alphafold2. The output was visualized in PyMol, where the designed structures were verified by alignment to the native structure.

### List of reference.

1. Lanzavecchia, A., Frühwirth, A., Perez, L., and Corti, D. (2016). Antibody-guided vaccine design: identification of protective epitopes. Curr Opin Immunol 41, 62-67. 10.1016/j.coi.2016.06.001.
2. Anasir, M.I., and Poh, C.L. (2019). Structural Vaccinology for Viral Vaccine Design. Front Microbiol 10, 738. 10.3389/fmicb.2019.00738.
3. Mba, I.E., Sharndama, H.C., Anyaegbunam, Z.K.G., Anekpo, C.C., Amadi, B.C., Morumda, D., Doowuese, Y., Ihezuo, U.J., Chukwukelu, J.U., and Okeke, O.P. (2023). Vaccine development for bacterial pathogens: Advances, challenges and prospects. Trop Med Int Health 28, 275-299. 10.1111/tmi. 13865.
4. Pushparaj, P., Nicoletto, A., Sheward, D.J., Das, H., Dopico, X.C., Vidakovics, L.P., Hanke, L., Chernyshev, M., Narang, S., Kim, S., et al. (2023). Immunoglobulin germline gene polymorphisms influence the function of SARS-CoV-2 neutralizing antibodies. Immunity 56, 193-206.e7. 10.1016/j.immuni.2022.12.005.
5. Hanke, L., Sheward, D.J., Pankow, A., Vidakovics, L.P., Karl, V., Kim, C., Urgard, E., Smith, N.L., Astorga-Wells, J., Ekström, S., et al. (2022). Multivariate mining of an alpaca immune repertoire identifies potent cross-neutralizing SARS-CoV-2 nanobodies. Sci Adv 8, eabm0220. 10.1126/sciadv.abm0220.
6. Ständer, S., Grauslund, L.R., Scarselli, M., Norais, N., and Rand, K. (2021). Epitope Mapping of Polyclonal Antibodies by Hydrogen-Deuterium Exchange Mass Spectrometry (HDX-MS). Anal Chem 93, 11669-11678. 10.1021/acs.analchem.1c00696.
7. Yu, C., and Huang, L. (2018). Cross-Linking Mass Spectrometry: An Emerging Technology for Interactomics and Structural Biology. Anal Chem 90, 144-165. 10.1021/acs.analchem.7b04431.
8. Mintseris, J., and Gygi, S.P. (2020). High-density chemical cross-linking for modeling protein interactions. Proc National Acad Sci 117, 93-102. 10.1073/pnas.1902931116.
9. Hauri, S., Khakzad, H., Happonen, L., Teleman, J., Malmström, J., and Malmström, L. (2019). Rapid determination of quaternary protein structures in complex biological samples. Nat Commun 10, 192. 10.1038/s41467-018-07986-1.
10. Bahnan, W., Happonen, L., Khakzad, H., Ahnlide, V.K., Neergaard, T., Wrighton, S., André, O., Bratanis, E., Tang, D., Hellmark, T., et al. (2022). A human monoclonal antibody bivalently binding two different epitopes in streptococcal M protein mediates immune function. Embo Mol Med. 10.15252/emmm.202216208.
11. Chiarot, E., Faralla, C., Chiappini, N., Tuscano, G., Falugi, F., Gambellini, G., Taddei, A., Capo, S., Cartocci, E., Veggi, D., et al. (2013). Targeted Amino Acid Substitutions Impair Streptolysin O Toxicity and Group A Streptococcus Virulence. Mbio 4, e00387-12. 10.1128/mbio.00387-12.
12. Pee, K. van, Neuhaus, A., D'Imprima, E., Mills, D.J., Kühlbrandt, W., and Yildiz, Ö. (2017). CryoEM structures of membrane pore and prepore complex reveal cytolytic mechanism of Pneumolysin. Elife 6, e23644. 10.7554/elife.23644.
13. Uchiyama, S., Döhrmann, S., Timmer, A.M., Dixit, N., Ghochani, M., Bhandari, T., Timmer, J.C., Sprague, K., Bubeck-Wardenburg, J., Simon, S.I., et al. (2015). Streptolysin O Rapidly Impairs Neutrophil Oxidative Burst and Antibacterial Responses to Group A Streptococcus. Front Immunol 6, 581. 10.3389/fimmu.2015.00581.
14. Azuar, A., Jin, W., Mukaida, S., Hussein, W.M., Toth, I., and Skwarczynski, M. (2019). Recent Advances in the Development of Peptide Vaccines and Their Delivery Systems against Group A Streptococcus. Nato Adv Sci Inst Se 7, 58. 10.3390/vaccines7030058.
15. Limbago, B., Penumalli, V., Weinrick, B., and Scott, J.R. (2000). Role of Streptolysin O in a Mouse Model of Invasive Group A Streptococcal Disease. Infect Immun 68, 6384-6390. 10.1128/iai.68.11.6384-6390.2000.
16. Walkinshaw, D.R., Wright, M.E.E., Mullin, A.E., Excler, J.-L., Kim, J.H., and Steer, A.C. (2023). The Streptococcus pyogenes vaccine landscape. Npj Vaccines 8, 16. 10.1038/s41541-023-00609-x.
17. Farrand, A.J., LaChapelle, S., Hotze, E.M., Johnson, A.E., and Tweten, R.K. (2010). Only two amino acids are essential for cytolytic toxin recognition of cholesterol at the membrane surface. Proc National Acad Sci 107, 4341-4346. 10.1073/pnas.0911581107.
18. Kucinskaite-Kodze, I., Simanavicius, M., Dapkunas, J., Pleckaityte, M., and Zvirbliene, A. (2020). Mapping of Recognition Sites of Monoclonal Antibodies Responsible for the Inhibition of Pneumolysin Functional Activity. Biomol 10, 1009. 10.3390/biom10071009.
19. Graaf, S.C. de, Hoek, M., Tamara, S., and Heck, A.J.R. (2022). A perspective toward mass spectrometry-based de novo sequencing of endogenous antibodies. Mabs 14, 2079449. 10.1080/19420862.2022.2079449.
20. Evans, R., O'Neill, M., Pritzel, A., Antropova, N., Senior, A., Green, T., Žídek, A., Bates, R., Blackwell, S., Yim, J., et al. (2022). Protein complex prediction with AlphaFold-Multimer. Biorxiv, 2021.10.04.463034. 10.1101/2021.10.04.463034.
21. Senior, A.W., Evans, R., Jumper, J., Kirkpatrick, J., Sifre, L., Green, T., Qin, C., Žídek, A., Nelson, A.W.R., Bridgland, A., et al. (2020). Improved protein structure prediction using potentials from deep learning. Nature 577, 706-710. 10.1038/s41586-019-1923-7.
22. Ruffolo, J.A., Chu, L.-S., Mahajan, S.P., and Gray, J.J. (2023). Fast, accurate antibody structure prediction from deep learning on massive set of natural antibodies. Nat Commun 14, 2389. 10.1038/s41467-023-38063-x.
23. Yin, R., Feng, B.Y., Varshney, A., and Pierce, B.G. (2022). Benchmarking AlphaFold for protein complex modeling reveals accuracy determinants. Protein Sci 31, e4379. 10.1002/pro.4379.
24. Petrotchenko, E.V., and Borchers, C.H. (2022). Protein Chemistry Combined with Mass Spectrometry for Protein Structure Determination. Chem Rev 122, 7488-7499. 10.1021/acs.chemrev.1c00302.
25. Masson, G.R., Jenkins, M.L., and Burke, J.E. (2017). An overview of hydrogen deuterium exchange mass spectrometry (HDX-MS) in drug discovery. Expert Opin Drug Dis 12, 981-994. 10.1080/17460441.2017.1363734.
26. Merkley, E.D., Rysavy, S., Kahraman, A., Hafen, R.P., Daggett, V., and Adkins, J.N. (2014). Distance restraints from crosslinking mass spectrometry: Mining a molecular dynamics simulation database to evaluate lysine-lysine distances. Protein Sci 23, 747-759. 10.1002/pro.2458.
27. Feil, S.C., Ascher, D.B., Kuiper, M.J., Tweten, R.K., and Parker, M.W. (2014). Structural Studies of Streptococcus pyogenes Streptolysin O Provide Insights into the Early Steps of Membrane Penetration. J Mol Biol 426, 785-792. 10.1016/j.jmb.2013.11.020.
28. Beslic, D., Tscheuschner, G., Renard, B.Y., Weller, M.G., and Muth, T. (2022). Comprehensive evaluation of peptide de novo sequencing tools for monoclonal antibody assembly. Brief Bioinform 24, bbac542. 10.1093/bib/bbac542.
29. Ambrosetti, F., Olsen, T.H., Olimpieri, P.P., Jiménez-García, B., Milanetti, E., Marcatilli, P., and Bonvin, A.M.J.J. (2020). proABC-2: PRediction Of AntiBody Contacts v2 and its application to information-driven docking. Bioinformatics 36, btaa644. 10.1093/bioinformatics/btaa644.
30. Chen, Z.-L., Meng, J.-M., Cao, Y., Yin, J.-L., Fang, R.-Q., Fan, S.-B., Liu, C., Zeng, W.-F., Ding, Y.-H., Tan, D., et al. (2019). A high-speed search engine pLink 2 with systematic evaluation for proteome-scale identification of cross-linked peptides. Nat Commun 10, 3404. 10.1038/s41467-019-11337-z.
31. Kolbowski, L., Combe, C., and Rappsilber, J. (2018). xiSPEC: web-based visualization, analysis and sharing of proteomics data. Nucleic Acids Res. 46, gky353-. 10.1093/nar/gky353.
32. Zundert, G.C.P. van, Trellet, M., Schaarschmidt, J., Kurkcuoglu, Z., David, M., Verlato, M., Rosato, A., and Bonvin, A.M.J.J. (2017). The DisVis and PowerFit Web Servers: Explorative and Integrative Modeling of Biomolecular Complexes. J Mol Biol 429, 399-407. 10.1016/j.jmb.2016.11.032.
33. Høie, M.H., Kiehl, E.N., Petersen, B., Nielsen, M., Winther, O., Nielsen, H., Hallgren, J., and Marcatili, P. (2022). NetSurfP-3.0: accurate and fast prediction of protein structural features by protein language models and deep learning. Nucleic Acids Res 50, gkac439-. 10.1093/nar/gkac439.
34. Vries, S.J. de, Dijk, M. van, and Bonvin, A.M.J.J. (2010). The HADDOCK web server for data-driven biomolecular docking. Nat Protoc 5, 883-897. 10.1038/nprot.2010.32.
35. Ambrosetti, F., Jiménez-García, B., Roel-Touris, J., and Bonvin, A.M.J.J. (2020). Modeling Antibody-Antigen Complexes by Information-Driven Docking. Structure 28, 119-129.e2. 10.1016/j.str.2019.10.011.
36. Moreira, I.S., Koukos, P.I., Melo, R., Almeida, J.G., Preto, A.J., Schaarschmidt, J., Trellet, M., Gümü 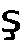 , Z.H., Costa, J., and Bonvin, A.M.J.J. (2017). SpotOn: High Accuracy Identification of Protein-Protein Interface Hot-Spots. Sci. Reports 7, 8007. 10.1038/s41598-017-08321-2.

## Claims

1. A Streptolysin O (SLO) epitope variant comprising the two discontinuous parts of Domain 3 linked together by a linker polypeptide replacing the extended loop part of Domain 1 and causing the SLO epitope variant to fold into substantially the same quaternary structure as the SLO epitope in *Streptococcus pyogenes* serotype M3 (strain SSI-1).

2. The SLO epitope variant according to claim 1, having the structure :
X1 - L1 - X2,
wherein X1 is the first discontinuous part of Domain 3, L1 is the linker polypeptide replacing the extended loop part of Domain 1, and X2 is the second discontinuous part of Domain 3.

3. The SLO epitope variant according to any one of claims 1-2, wherein said linker polypeptide (L1) replacing the extended loop part of Domain 1 has a length of less than 12 amino acid residues.

4. The SLO epitope variant according to any one of claims 1-3, wherein said linker polypeptide (L1) replacing the Extended loop part of Domain 1 (L1) is selected from the group consisting of APNG, APGG, CPNG and CPGG.

5. The SLO epitope variant according to any of claims 1-4, wherein said first discontinuous part of Domain 3 (X1) has at least 80%, at least 87%, at least 89%, at least 93% or at least 95% amino acid identity to the polypeptide SEQ ID NO: 3.

6. The SLO epitope variant according to any one of claims 1-5, wherein said second discontinuous part of Domain 3 (X2) has at least 81%, at least 85%, at least 89%, at least 93% or at least 97% amino acid identity to the polypeptide SEQ ID NO: 5.

7. An immunogenic protein comprising a SLO epitope variant as defined in any one of claims 1-6.

8. A fusion protein comprising at least one SLO epitope variant as defined in any one of claims 1-6 and at least one nanoparticle trimeric component.

9. A protein nanoparticle comprising a SLO epitope variant as defined in any one of claims 1-6.

10. A protein nanoparticle comprising a fusion protein as defined in claim 8.

11. An antibody binding to a SLO epitope variant as defined in any one of claims 1-6.

12. A nucleic acid molecule encoding a SLO epitope variant, an immunogenic protein, a fusion protein, a protein nanoparticle or an antibody as defined in any one of claims 1-11.

13. A pharmaceutical composition comprising a SLO epitope variant, an immunogenic protein, a fusion protein, a protein nanoparticle or an antibody as defined in any one of claims 1-11, and a pharmaceutically acceptable excipient.

14. A composition comprising a SLO epitope variant, an immunogenic protein, a fusion protein, a protein nanoparticle or an antibody as defined in any one of claims 1-11, for use in the treatment or prophylactics of a S. *pyogenes* associated disease, disorder or condition.

15. A method for the treatment or prophylactics of a S. *pyogenes* associated disease, disorder or condition comprising administering a therapeutically effective amount of a SLO epitope variant, an immunogenic protein, a fusion protein, a protein nanoparticle, an antibody or a pharmaceutical composition as defined in any one of claims 1-11 and 13-14.
